# EUROPEAN PATENT SPECIFICATION

(11) **EP 3 922 987 B1**
(45) Date of publication and mention of the grant of the patent: **27.08.2025**
(21) Application number: 21174697.9
(22) Date of filing: 19.05.2021
(51) Int. Cl.: G01N 27/12, B82Y 15/00, G01N 33/00

(54) **ACETONE GAS SENSOR USING NB-DOPED WO3 AND FABRICATION METHOD THEREOF**
ACETONGASSENSOR MIT NB-DOTIERTER WO3 NANOSTRUKTUR UND DESSEN HERSTELLUNGSVERFAHREN
CAPTEUR DE GAZ ACÉTONE UTILISANT UNE NANOSTRUCTURE DE WO3 DOPÉ AU NB ET SON PROCÉDÉ DE FABRICATION

(30) Priority: 12.06.2020 KR 20200071502
(43) Date of publication of application: 15.12.2021
(73) Proprietor: Korea University Research and Business Foundation, Seoul (KR)
(72) Inventor: Lee, Jong Heun, 05649 Seoul (KR); Choi, Hun Ji, 21685 Incheon (KR); Yoon, Ji Wook, 54969 Jeollabuk-do (KR)
(74) Representative: RGTH

(56) References cited:
- KR-B1- 101 766 334
- GAURY J. ET AL: "Characterization of Nb-doped WO3 thin films produced by Electrostatic Spray Deposition", THIN SOLID FILMS, vol. 534, 31 January 2013 (2013-01-31), AMSTERDAM, NL, pages 32 - 39, XP055842294, ISSN: 0040-6090, DOI: 10.1016/j.tsf.2013.01.080
- YAN AIHUA ET AL: "An efficient method to modulate the structure, morphology and properties of WO3through niobium doping", JOURNAL OF ALLOYS AND COMPOUNDS, ELSEVIER SEQUOIA, LAUSANNE, CH, vol. 610, 6 May 2014 (2014-05-06), pages 132 - 137, XP028874454, ISSN: 0925-8388, DOI: 10.1016/J.JALLCOM.2014.04.188
- ZAPPA DARIO: "The Influence of Nb on the Synthesis of WO3 Nanowires and the Effects on Hydrogen Sensing Performance", SENSORS, vol. 19, no. 10, 20 May 2019 (2019-05-20), pages 2332, XP055842288, DOI: 10.3390/s19102332
- LIAO WEN LING: "Processing and Characterization of [epsilon]-WO3 Processed by Flame Spray Pyrolysis", MASTER THESIS, 31 December 2014 (2014-12-31), XP055842851, Retrieved from the Internet <URL:https://ir.stonybrook.edu/xmlui/bitstream/handle/11401/76328/Liao_grad.sunysb_0771M_12158.pdf?sequence=1> [retrieved on 20210920]
- SUVARNA R BATHE ET AL: "Influence of Nb doping on the electrochromic properties of WO3 films", JOURNAL OF PHYSICS D: APPLIED PHYSICS, INSTITUTE OF PHYSICS PUBLISHING LTD, GB, vol. 40, no. 23, 7 December 2007 (2007-12-07), pages 7423 - 7431, XP020126807, ISSN: 0022-3727
- KIM BO-YOUNG ET AL: "Dual Role of Multiroom-Structured Sn-Doped NiO Microspheres for Ultrasensitive and Highly Selective Detection of Xylene", APPLIED MATERIALS & INTERFACES, vol. 10, no. 19, 16 May 2018 (2018-05-16), US, pages 16605 - 16612, XP055843029, ISSN: 1944-8244, Retrieved from the Internet <URL:https://pubs.acs.org/doi/pdf/10.1021/acsami.8b02412> DOI: 10.1021/acsami.8b02412
- CHOI HUN JI ET AL: "Highly selective, sensitive, and rapidly responding acetone sensor using ferroelectric [epsilon]-WO3 spheres doped with Nb for monitoring ketogenic diet efficiency", SENSORS AND ACTUATORS B: CHEMICAL, ELSEVIER BV, NL, vol. 338, 19 March 2021 (2021-03-19), XP086541165, ISSN: 0925-4005, [retrieved on 20210319], DOI: 10.1016/J.SNB.2021.129823
- WANG: "Ferroelectric WO3 nanoparticles for acetone selective detection", CHEMISTRY OF MATERIALS, vol. 20, no. 15, 1 January 2008 (2008-01-01), pages 4794 - 4796, XP055000062, ISSN: 0897-4756, DOI: 10.1021/cm800761e
- WANG L: "Supporting Information", 4 July 2008 (2008-07-04), XP093130783, Retrieved from the Internet <URL:https://pubs.acs.org/doi/suppl/10.1021/cm800761e/suppl_file/cm800761e-file002.pdf> [retrieved on 20240213]

## Description

### BACKGROUND

Embodiments of the inventive concept described herein relate to an acetone gas sensor using ferroelectric Nb-doped WO₃ and a fabrication method thereof.
J. Gaury et al. "Characterization of Nb-doped WO3 thin films produced by Electrostatic Spray Deposition" discloses a characterization of Nb·-doped W01 thin films produced by Electrostatic Spray Deposition on glass substrates. The films were produced by electrospraying a tungsten isopropoxide in 2-propanol precursor solution in a temperature-·controlled environment. Film morphologies and crystal structures were characterized by Scanning Electron Microscopy, Transmission Electron Microscopy, and X-Ray Diffraction, whereas band gap values. composition, and oxidation state of the materials were determined by Ultraviolet-Visible Spectroscopy Energy Dispersive X-Ray, and X-Ray Photoemission Spectroscopy, respectively. It is shown that highly-porous tree-like morphologies can be achieved by adjusting the deposition temperature to 325 °C. Under these conditions, the size of the primary particles of the films ranges from 150 to 200 nm, and their crystallinity can be varied from completely amorphous for the as-deposited samples to hexagonal structure, for the annealed samples. The measured Nb content of the doped samples was found to be similar to that of the precursor solution, leading to an increase of the band gap of 0.23 eV as compared to the pure WO3 sample.
Aihua Yan et al. "An efficient method to modulate the structure, morphology and properties of WO3 through niobium doping" discloses that Nb-doped WO3 was synthesized to investigate the effect of doping on composition, structure and optical properties. It is shown that niobium ion implantation in WO3 structure results in the morphological evolution from nanosheet into nanoparticle. 5 at% niobium ion doping induces the phase transformation from monoclinic y-WO3 to monoclinic ε-WO3. UV-vis spectrum shows that niobium implantation results in the increase of the band gap and the shift of absorption edge. Photoelectric measurement indicates that photocurrent increases firstly and then decreases under UV irradiation. The results reported in this study will be useful in formulating optimum doping process to enhance desired photoelectric properties.
In Dario Zappa "The influence of Nb on the Synthesis of WO₃ Nanowires and the Effects on Hydrogen Sensing Performance" the influence of Nb on the sensing performances of WO3 nanowires (NWs) synthetized by a low-cost thermal oxidation method is investigated. The morphology and the structure of these Nb-WO3 nanowires were investigated by field emission scanning electron microscope (FE-SEM), high-resolution transmission electron microscope (HR-TEM), X-ray diffraction (XRD), Raman and X-ray photoelectron (XPS) spectroscopies, confirming that the addition of Nb does not modify significantly the monoclinic crystal structure of WO3. Moreover, these NWs have been integrated into chemical sensors, and their performances toward hydrogen and some common interfering compounds has been assessed.
Wen Ling Liao "Processing and Characterization of ε-WO₃ Processed by Flame Spray Pyrolysis" focuses on using flame spray pyrolysis to produce pure ε -WO3 nanoparticles that can be stable at high temperatures. The advantage of flame spray pyrolysis is that it provides large temperature gradient for the product to nucleate and short residence time for its sintering; therefore, the metastable phase, ε -WO3, could be synthesized directly from a range of precursors (solutes) in a single step, by increasing the nucleation rate and reducing the residence time.
Wang et al.: "Ferroelectric WO3 nanoparticles for acetone selective detection" with corresponding "Supporting Information" discloses how ε-WO3 nanoparticles are produced by :flame spray pyrolysis. Cr doping is able to stabilize this acentric structure during heat treatment. A 10 atom % Cr-doped WO3 based chemi-resistive sensor is produced which shows fast, stable, fairly sensitive and highly selective response to low concentrations of acetone.

The present invention is derived from a study (Detailed task number: 2020R1A2C300893311, Research management institution: Korea Research Foundation, Project Title: Development of low molecular weight gas high selectivity detection sensor and high efficiency photoelectrode for water decomposition through functional oxide-MOF heterojunction design, Organizer: Korea University Industry, Research Period: 2020.03.01 ~ 2025.02.28, Contribution Rate: 1) conducted as part of a 'Mid-sized researcher support project (Type 2)' of the ICT in Korea. There is no property interest of the Korean government in any aspect of this invention.

An oxide semiconductor type gas sensor has various advantages such as high sensitivity, fast response speed, high reliability, economical price, and ease of integration into mobile and small devices, thereby being used in a variety of applications such as explosive gas detection, driver drinking measurement, industrial hazardous gas detection, food freshness evaluation, vehicle interior and indoor/outdoor environmental gas monitoring. With the recent advancement of the industry, the development of the 4th industrial revolution, and increasing interest in human health and environmental pollution, a demand for gas sensors such as indoor and outdoor hazardous environment gas detection sensor, non-invasive disease diagnosis gas sensor, ultra-compact artificial olfactory sensor mounted on mobile devices increases rapidly. In particular, for new applications of gas sensors, a demand for gas sensors exhibiting high sensitivity and high selective detection characteristics greatly increases.

When a disease progresses, a gas that comes out of human breath as a metabolic process is called a biomarker gas, and non-invasive disease diagnosis is possible through detection and concentration of the biomarker gas. Representative biomarker gases include hydrogen, ammonia, toluene, and carbon monoxide, and additionally, ethanol is included in human breath. Acetone is a biomarker gas that occurs in excess in breathing of people with type 1 diabetes. In particular, detection of exhaled acetone is a very important task for an effective test of insulin-dependent diabetic ketoacidosis, which occurs in excess of ketones due to insufficient insulin. In addition, in the breath of people on a ketogenic diet, who use a diet of low carbohydrate and high fat consumption, acetone is measured in excess, and body fat decomposition rate and a ketogenic diet effect may be found from the measured acetone concentration.

Considering that a person's expiration is more than 80% relative humidity, there is a need for a sensitive material capable sensing low concentration acetone gas in highly sensitive and selective manner in a high humidity atmosphere. However, there is a need for a sensitive material capable of selectively detecting acetone, because most of the oxide semiconductor gas sensors currently used show similar sensitivity to other biomarker gases, or high sensitivity only to highly reactive gases such as ethanol. Further, the oxide semiconductor type gas sensor decreases gas sensitivity in a humid atmosphere, and thus it is a very difficult technical task to detect a low concentration of acetone in a high humidity atmosphere with high sensitivity.

### SUMMARY

It is the object of the present invention to provide an improved gas sensor for the detection of acetone gas and a fabrication method therefor.

This object is achieved by the subject-matter of the independent claims. Embodiments are defined by the dependent claims.

Any embodiment described in the description that is not covered by the claims is for illustrative purposes only.

Claim 1 defines a a gas sensor for selective detection of acetone gas capable of highly sensitive and selectively detecting acetone among gases from human breath.

Claim 2 defines a method of manufacturing a gas sensor including changing a composition of a substance constituting a gas sensitizing layer and selectively reacting to acetone in a humidity atmosphere.

Meanwhile, the technical problems to be achieved in the inventive concept are not limited to the technical problems mentioned above, and other technical problems not mentioned will be clearly understood by those of ordinary skill in the art from the following description.

### BRIEF DESCRIPTION OF THE FIGURES

The above and other objects and features will become apparent from the following description with reference to the following figures, wherein like reference numerals refer to like parts throughout the various figures unless otherwise specified, and wherein:
FIGS. 1 and 2 are cross-sectional views schematically illustrating an exemplary gas sensor;
FIG. 3 illustrates a flow chart illustrating an exemplary method of manufacturing a gas sensor;
FIG. 4 illustrates SEM images of spherical nanostructures according to Comparative Examples and Example (spherical nanostructures are not in accordance with the present invention);
FIG. 5 illustrates SEM images of egg-yolk nanostructures according to Comparative Examples and Examples of the inventive concept;
FIG. 6 illustrates TEM images and elemental analysis images of nanostructures according to Comparative Examples and Examples (not according to the present invention);
FIGS. 7 and 8 illustrate graphs illustrating results of X-ray diffraction analysis of nanostructures according to Comparative Examples and Examples (the EXAMPLES in FIG. 7 are not according to the present invention, EXAMPLE 2-1 in FIG. 8 is according to the present invention);
FIG. 9 illustrates graphs of gas responses to 1 ppm acetone, 1 ppm ethanol, 1 ppm carbon monoxide, 20 ppm hydrogen, 5 ppm ammonia and 50 ppb toluene at an operating temperature of 300 °C to 450 °C in gas sensors according to Comparative Examples and Examples (not according to the present invention);
FIGS. 10 and 11 illustrate graphs of gas responses to 1 ppm acetone, 1 ppm ethanol, 1 ppm carbon monoxide, and 1ppm hydrogen at an operating temperature of 375 °C in gas sensors according to Comparative Examples and Examples (the EXAMPLES in FIG. 10 are not according to the present invention, EXAMPLE 2-1 in FIG. 11 is according to the present invention);
FIG. 12 illustrates graphs of characteristics results for acetone gas concentrations (0.25 to 1 ppm) of gas sensors according to Example 1-3 (not according to the present invention) at an operating temperature of 375 °C; and
FIG. 13 illustrates graphs of characteristics results for repetitive acetone gas (1 ppm) of gas sensors according to Example 1-3 (not according to the present invention) at an operating temperature of 375 °C.

### DETAILED DESCRIPTION

Hereinafter, an embodiment of the inventive concept will be described in more detail with reference to the accompanying drawings. Embodiments of the inventive concept may be modified in various forms, and it should not be construed that the scope of the inventive concept is limited to the following examples. This embodiment is provided to more completely describe the inventive concept to those with average knowledge in the art. Therefore, a shape of an element in the drawings has been exaggerated to emphasize a clearer description.

A configuration of the inventive concept for clarifying the solution to the problem to be solved by the inventive concept will be described in detail with reference to the accompanying drawings based on a preferred embodiment of the inventive concept, and in assigning reference numbers to elements of the drawings, the same reference numbers were assigned to the same elements even when they are on different drawings. It should be noted in advance that components of other drawings may be cited if necessary when describing the drawings.

A gas sensor according to the inventive concept is a gas sensor including a gas sensitizing layer formed of tungsten oxide doped with niobium. Prior to the inventive concept, that niobium-doped tungsten oxide has a low gas sensitivity to interference gases such as ethanol, ammonia, hydrogen, carbon monoxide, toluene, or the like, and has a high gas sensitivity to acetone with high sensitivity and high selectivity, is unknown, and is uses and effects that are first found in the inventive concept.

In addition, the gas sensor according to the inventive concept is capable of performing non-invasive disease diagnosis through detection and concentration of biomarker gas from human breath in a simple and economical way because the gas sensor is a nanostructure sensitive material and is capable of selectively detecting acetone.

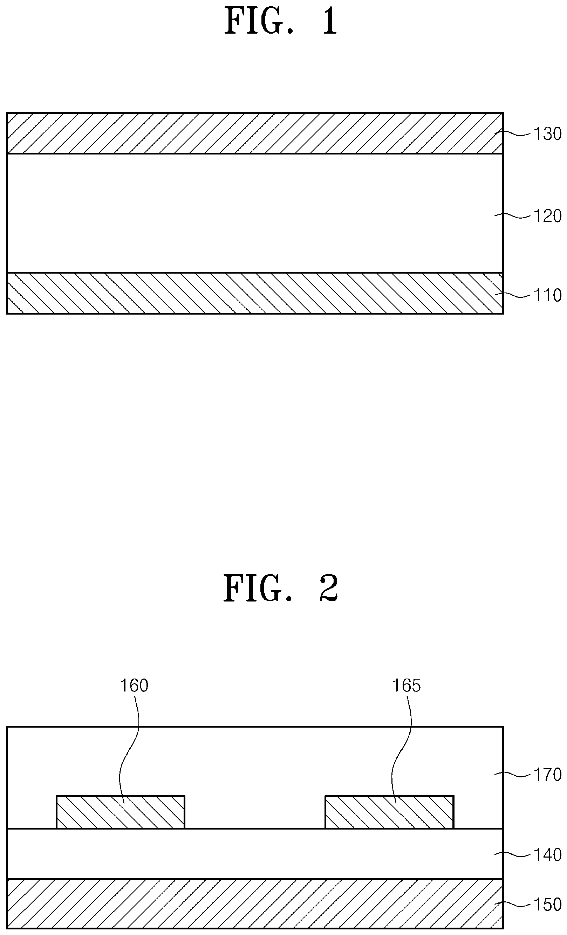

FIGS. 1 and 2 are cross-sectional views schematically illustrating an exemplary gas sensor.

The gas sensor illustrated in FIG. 1 is a structure in which electrodes 110 and 130 are provided on lower and upper surfaces of a gas sensitizing layer 120, respectively.

The gas sensor illustrated in FIG. 2 includes a structure, in which a microheater 150 is formed on a lower surface of a substrate 140, and two electrodes 160 and 165 are formed on an upper surface of the substrate 140, and a gas sensitizing layer 170 is formed thereon. The gas sensitizing layers 120 and 170 in the gas sensors of FIGS. 1 and 2 are all formed of tungsten oxide doped with niobium, and the amount of niobium may be adjusted when necessary.

Meanwhile, in the gas sensor it is preferable that a thickness of each of the gas sensitizing layers 120 and 170 is formed to be 2 µm to 40 µm.

Here, when the thickness of the gas sensitizing layers 120 and 170 is less than 2 µm, there is a problem of not exhibiting selectivity to acetone, and when the thickness of the gas sensitizing layers 120 and 170 exceeds 40 µm, there is a problem of largely decreasing the sensitivity.

In the inventive concept, niobium is replaced inside a tungsten oxide lattice by using a high solubility of tungsten oxide to niobium, to change a phase of tungsten oxide from a γ phase stable at room temperature to a ε phase stable at low temperature, thereby implementing a sensitizing material with ferroelectricity.

Here, unlike an interference gas, acetone having a relatively large dipole moment is better attracted by a dipole-dipole interaction, thereby improving gas sensitizing characteristics with a high sensitivity and high selectivity.

In the inventive concept, when a concentration ratio of niobium (Nb) and tungsten (W) is less than 2at% and when a concentration ratio exceeds 50at%, high sensitivity and high selectivity to acetone may not be secured.

Here, when a concentration ratio is lower than 2at%, sensitivity to acetone is low and selectivity of acetone to other gases is not secured. When a concentration ratio exceeds 50at%, selectivity of acetone to other gases is decreased and resistance of material becomes high (more than 1GΩ at 375 °C) as Nb goes into excess. That is, it is unsuitable to actual applications because more expensive equipment is required to measure this.

In the inventive concept, when niobium is added to a tungsten oxide sensitizing material having various structures, sensitivity and selectivity to acetone gas may be increased several times or more with high stability, regardless of the structure.

According to the inventive concept, the concentration of niobium may be adjusted and niobium having the adjusted concentration may be added to the tungsten oxide sensitizing material having various structures, thereby changing the particle phase of tungsten.

Meanwhile, the gas sensor may obtain a high selectivity to react only acetone gas. It is confirmed that the manufactured high sensitizing oxide semiconductor gas sensor may contribute to commercialization of gas sensors due to excellent long-term stability and selectivity.

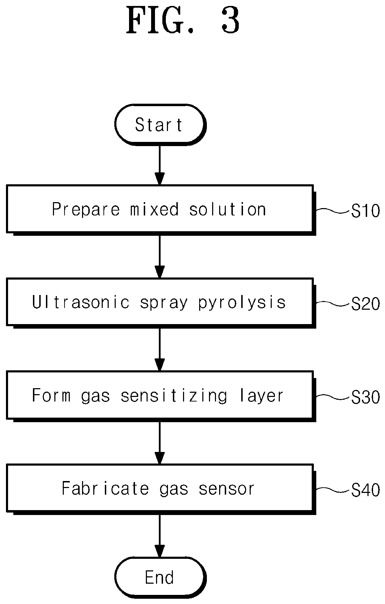

FIG. 3 illustrates a flow chart illustrating an exemplary method of manufacturing a gas sensor.

First, after adding niobium and tungsten to distilled water and stirring them, a mixed solution for spraying is prepared (operation S10).

Here, a concentration ratio of niobium (Nb) and tungsten (W) contained in 100 mL of distilled water is preferably 2at% to 50at%.

Here, when the concentration ratio of niobium (Nb) and tungsten (W) is less than 2at% and when the concentration ratio exceeds 50at%, high sensitivity and high selectivity to acetone, and measurable sensor resistance may not be simultaneously secured.

When the concentration ratio is lower than 2at%, the sensitivity to acetone is also low and selectivity of acetone to other gases is not secured. When the concentration ratio exceeds 50at%, the selectivity of acetone to other gases is decreased and resistance of material becomes high (more than 1GΩ at 375 °C) as Nb goes into excess. That is, it is unsuitable to actual applications because more expensive equipment is required to measure this.

Next, the mixed solution is subjected to ultrasonic spray pyrolysis to form a precursor, and the precursor is heat-treated to form a nanostructure (operation S20).

The nanostructure is formed through a process using the mixed solution containing niobium and tungsten. For example, the mixed solution may be sprayed through ultrasonic waves and then thermally decomposed by passing through a quartz tube, to form an Nb-doped WO₃ nanostructure.

This operation may include a process of generating fine droplets by spraying the mixed solution and introducing the fine droplets into the reactor to synthesize and recover powder. A size of each of the fine droplets may be adjusted by a pressure inside a spray device, a concentration of a spray solution, a viscosity of the spray solution, and the like. By heating in a reactor, most of the solvent contained in the fine droplets is volatilized, and a polymer precursor, oxide, and a metal catalyst precursor component remain.

A gas sensitizing layer is formed using the nanostructure (operation S30) to fabricate the gas sensor (operation S40).

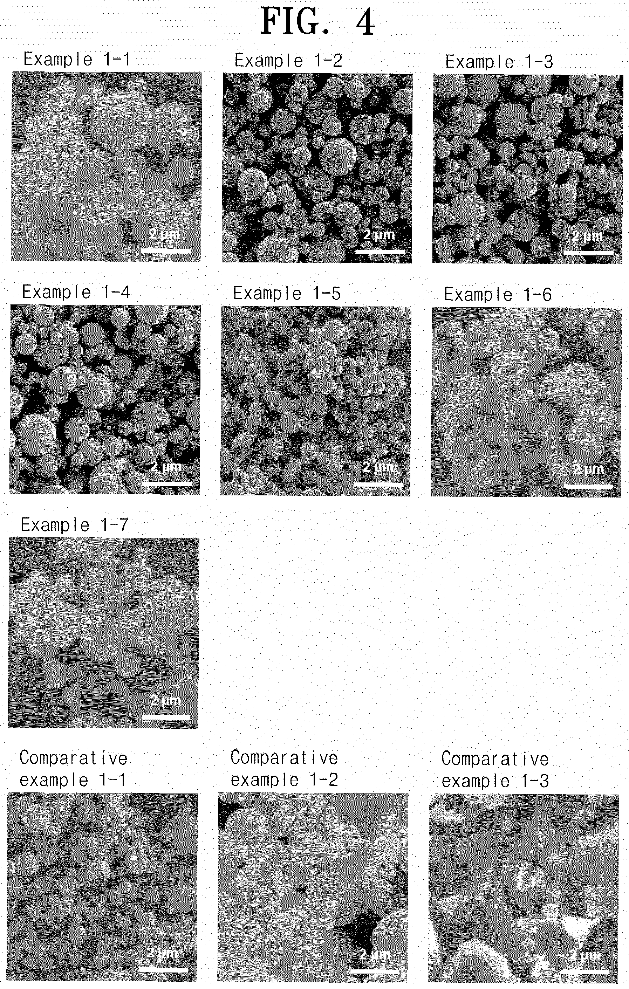

Hereinafter, referring to Table 1 and FIGS. 4 to 13, by using an ultrasonic spray pyrolysis method, gas sensors of Examples including a nanostructure manufactured by adjusting a doping concentration ratio of niobium (Nb) and tungsten (W), and gas sensors of Comparative Examples will be compared and Examples 1-1 to 1-7 are not in accordance with the present invention, Example 2-1 is in accordance with the present invention.

**[Table 1]**

| | Particle structure | Concentration ratio (at%) (Nb/(Nb+W)) | Synthetic method |
|---|---|---|---|
| Example 1-1 | Spherical shape | 2 | ultrasonic spray pyrolysis (Nb+W) |
| Example 1-2 | Spherical shape | 5 | ultrasonic spray pyrolysis (Nb+W) |
| Example 1-3 | Spherical shape | 10 | ultrasonic spray pyrolysis (Nb+W) |
| Example 1-4 | Spherical shape | 15 | ultrasonic spray pyrolysis (Nb+W) |
| Example 1-5 | Spherical shape | 20 | ultrasonic spray pyrolysis (Nb+W) |
| Example 1-6 | Spherical shape | 30 | ultrasonic spray pyrolysis (Nb+W) |
| Example 1-7 | Spherical shape | 40 | ultrasonic spray pyrolysis (Nb+W) |
| Comparative Example 1-1 | Spherical shape | 0(Nb 0%) | ultrasonic spray pyrolysis (Nb+W) |
| Comparative Example 1-2 | Spherical shape | 100(Nb 100%) | ultrasonic spray pyrolysis (Nb+W) |
| Comparative Example 1-3 | Spherical shape | 10 | after ultrasonic spray pyrolysis (W alone), Nb addition liquid reaction |
| Example 2-1 | Egg yolk | 10 | ultrasonic spray pyrolysis (Nb+W) |
| Comparative Example 2-1 | Egg yolk | 0(Nb 0%) | ultrasonic spray pyrolysis (Nb+W) |

### <Example 1-1> (not in accordance with the present invention)

As a concentration ratio of Nb/(Nb+W) was calculated to be 0.02, 2.41 g of ammonium metatungstate hydrate [(NH₄)6H₂W₁₂O₄₀.xH₂O, 99.99%, Sigma-Aldrich, USA] and 0.06 g of ammonium niobate(V) oxalate hydrate [C₄H₄NNbO₉.xH₂O, 99,99%, Sigma-Aldrich, USA] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form an Nb-doped WO₃ spherical structure. The obtained heat-treated Nb-doped WO₃ structure powder was mixed with an organic binder, and a gas sensitizing layer was applied on an alumina substrate with two electrodes. Here, coating was used in a sense that includes various methods such as printing, brushing, blade coating, dispensing, and micro pipette dropping.

Then, heat treatment at 450 °C for 2 hours was performed to remove a solvent, thereby forming a gas sensor. The manufactured gas sensor was placed inside a gas sensing chamber composed of a quartz tube, and pure air or mixed gas was alternately injected to measure the change in resistance of the gas sensor in real time. After mixing gases to an appropriate concentration through MFC in advance, the gases were rapidly injected using a 4-way valve to change the gas concentration inside the gas sensing chamber. The total flow rate inside the gas sensing chamber was fixed at 200 sccm, and thus a temperature of the gas sensor was maintained even with rapid changes in gas concentration.

### <Example 1-2> (not in accordance with the present invention)

As a concentration ratio of Nb/(Nb+W) was calculated to be 0.05, 2.34 g of ammonium metatungstate hydrate [(NH₄)6H₂W₁₂O₄₀.xH₂O, 99.99%, Sigma-Aldrich, USA] and 0.15 g of ammonium niobate(V) oxalate hydrate [C₄H₄NNbO₉.xH₂O, 99,99%, Sigma-Aldrich, USA] were added to 100 mL distilled water and were stirred until all the reagents were dissolved, thereby preparing a spray solution. The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form an Nb-doped WO₃ spherical structure.

Then, the method of manufacturing the sensor and the gas sensitization measurement were the same as in Example 1-1.

### <Example 1-3> (not in accordance with the present invention)

As a concentration ratio of Nb/(Nb+W) was calculated to be 0.1, 2.22 g of ammonium metatungstate hydrate [(NH₄)6H₂W₁₂O₄₀.xH₂O, 99.99%, Sigma-Aldrich, USA] and 0.30 g of ammonium niobate(V) oxalate hydrate [C₄H₄NNbO₉.xH₂O, 99,99%, Sigma-Aldrich, USA] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (900 °C) connected to a spray outlet, thereby preparing a precursor. Then, the prepared precursor was heat-treated at 600 °C for 2 hours, to form an Nb-doped WO₃ spherical structure.

Then, the method of manufacturing the sensor and the gas sensitization measurement were the same as in Example 1-1.

### <Example 1-4> (not in accordance with the present invention)

As a concentration ratio of Nb/(Nb+W) was calculated to be 0.15, 2.09 g of ammonium metatungstate hydrate [(NH₄)6H₂W₁₂O₄₀.xH₂O, 99.99%, Sigma-Aldrich, USA] and 0.45 g of ammonium niobate(V) oxalate hydrate [C₄H₄NNbO₉.xH₂O, 99,99%, Sigma-Aldrich, USA] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form an Nb-doped WO₃ spherical structure.

Then, the method of manufacturing the sensor and the gas sensitization measurement were the same as in Example 1-1.

### <Example (not in accordance with the present invention)

As a concentration ratio of Nb/(Nb+W) was calculated to be 0.2, 1.97 g of ammonium metatungstate hydrate [(NH₄)6H₂W₁₂O₄₀.xH₂O, 99.99%, Sigma-Aldrich, USA] and 0.61 g of ammonium niobate(V) oxalate hydrate [C₄H₄NNbO₉.xH₂O, 99,99%, Sigma-Aldrich, USA] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form an Nb-doped WO₃ spherical structure.

Then, the method of manufacturing the sensor and the gas sensitization measurement were the same as in Example 1-1.

### <Example 1-6> (not in accordance with the present invention)

As a concentration ratio of Nb/(Nb+W) was calculated to be 0.3, 1.72 g of ammonium metatungstate hydrate [(NH₄)6H₂W₁₂O₄₀.xH₂O, 99.99%, Sigma-Aldrich, USA] and 0.9 g of ammonium niobate(V) oxalate hydrate [C₄H₄NNbO₉.xH₂O, 99,99%, Sigma-Aldrich, USA] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form an Nb-doped WO₃ spherical structure.

Then, the method of manufacturing the sensor and the gas sensitization measurement were the same as in Example 1-1.

### <Example (not in accordance with the present invention)

As a concentration ratio of Nb/(Nb+W) was calculated to be 0.4, 1.47 g of ammonium metatungstate hydrate [(NH₄)6H₂W₁₂O₄₀.xH₂O, 99.99%, Sigma-Aldrich, USA] and 1.21 g of ammonium niobate(V) oxalate hydrate [C₄H₄NNbO₉.xH₂O, 99,99%, Sigma-Aldrich, USA] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form an Nb-doped WO₃ spherical structure.

Then, the method of manufacturing the sensor and the gas sensitization measurement were the same as in Example 1-1.

### <Comparative Example 1-1>

2.46 g of ammonium metatungstate hydrate [(NH4)6H2W12O40·xH2O, 99.99%, Sigma-Aldrich, USA] was added to 100 mL distilled water and stirred until all the reagents were dissolved to prepare a spray solution.

The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form a WO₃ spherical structure.

Then, the method of manufacturing the sensor and the gas sensitization measurement were the same as in Example 1-1.

### <Comparative Example 1-2>

3.03 g of ammonium metatungstate hydrate [(NH4)6H2W12O40·xH2O, 99.99%, Sigma-Aldrich, USA] was added to 100 mL distilled water and stirred until all the reagents were dissolved to prepare a spray solution.

The prepared spray solution is ultrasonic sprayed in the air at a flow rate of 10 L·min⁻¹ while passing through a furnace (900 °C) connected to a spray outlet, thereby preparing a precursor.

Then, the prepared precursor was heat-treated at 600 °C for 2 hours, to form a WO₃ spherical structure.

Then, the method of manufacturing the sensor and the gas sensitization measurement were the same as in Example 1-1.

### <Comparative Example 1-3>

2.46 g of ammonium metatungstate hydrate [(NH4)6H2W12O40·xH2O, 99.99%, Sigma-Aldrich, USA] was added to 100 mL distilled water and stirred until all the reagents were dissolved to prepare a spray solution.

The prepared spray solution is ultrasonic sprayed in the air at a flow rate of 10 L·min⁻¹ while passing through a furnace (900 °C) connected to a spray outlet, thereby preparing a precursor. Then, the prepared precursor was heat-treated at 600 °C for 2 hours, to form a WO₃ spherical structure.

As a concentration ratio of Nb/(Nb+W) was calculated to be 0.1, 0.185g of WO₃ spherical structure, 0.0242g of ammonium niobate(V) oxalate hydrate [C₄H₄NNbO₉ .xH₂O, 99,99%, Sigma-Aldrich, USA], and 0.48g of urea [NH₂CONH₂, Junsei, 99.0%, Japan] were added to 100 mL distilled water and were stirred at 80 °C for 8 hours.

Then, the prepared precursor was heat-treated at 600 °C for 2 hours, to form an Nb-doped WO₃ spherical structure. Then, the method of manufacturing the sensor and the gas sensitization measurement were the same as in Example 1-1.

### <Example 2-1> (in accordance with the present invention)

As a concentration ratio of Nb/(Nb+W) was calculated to be 0.1, 2.22 g of ammonium metatungstate hydrate [(NH₄)6H₂W₁₂O₄₀.xH₂O, 99.99%, Sigma-Aldrich, USA], 0.30 g of ammonium niobate(V) oxalate hydrate [C₄H₄NNbO₉.xH₂O, 99,99%, Sigma-Aldrich, USA], and 1.5 g of dextrin [(C₆H₁₀O₅)x.xH₂O, Junsei, 99.0%, Korea] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (400 °C) connected to a spray outlet, thereby preparing a precursor.

Then, the prepared precursor was heat-treated at 600 °C for 2 hours, to form an Nb-doped WO₃ egg-yolk structure.

Then, the method of manufacturing the sensor and the gas sensitization measurement were the same as in Example 1-1.

### <Comparative Example 2-1>

2.46 g of ammonium metatungstate hydrate [(NH₄)6H₂W₁₂O₄₀.xH₂O, 99.99%, Sigma-Aldrich, USA] and 1.5 g of dextrin [(C₆H₁₀O₅)x.xH₂O, Samchun, Korea] were added to 100 mL distilled water and were stirred until all the reagents were dissolved, thereby preparing a spray solution.

The prepared spray solution is ultrasonic sprayed in the air at a flow rate of 10 L·min⁻¹ while passing through a furnace (400 °C) connected to a spray outlet, thereby preparing a precursor.

Then, the prepared precursor was heat-treated at 600 °C for 2 hours, to form a WO₃ egg-yolk structure.

Then, the method of manufacturing the sensor and the gas sensitization measurement were the same as in Example 1-1.

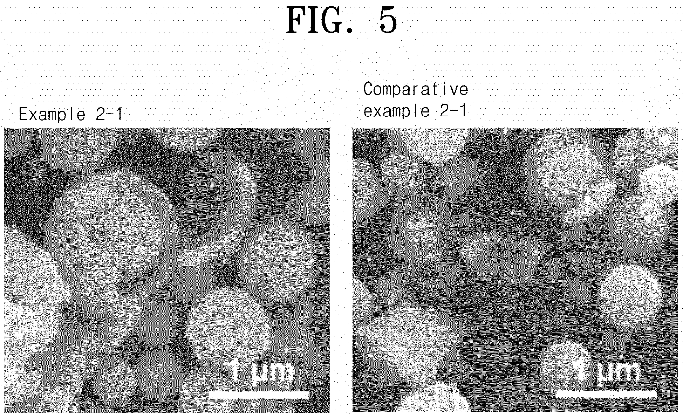

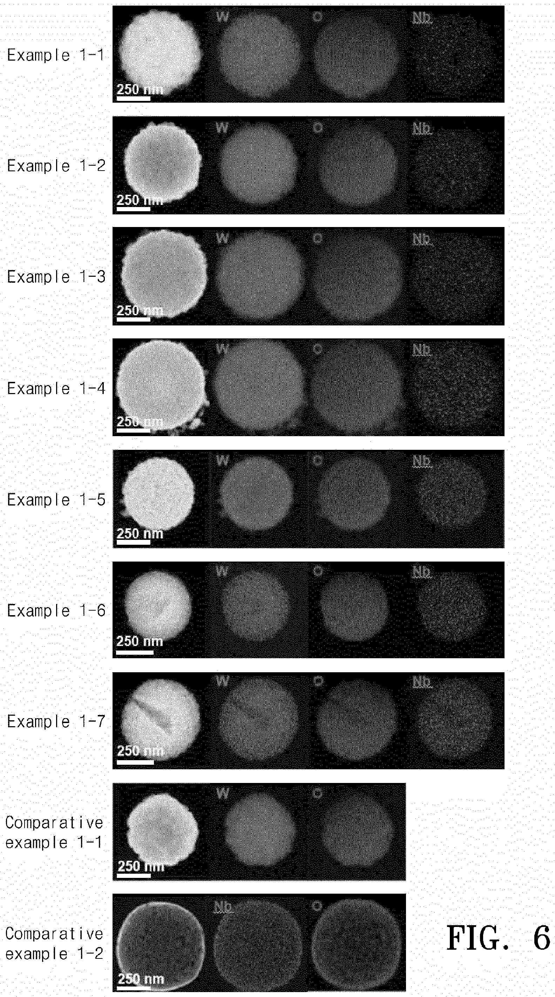

FIG. 4 illustrates SEM images of spherical nanostructures according to Comparative Examples and Examples (not in accordance with the present invention), FIG. 5 illustrates SEM images of egg-yolk nanostructures according to Comparative Examples and Examples of the inventive concept, and FIG. 6 illustrates TEM images and elemental analysis images of nanostructures according to Comparative Examples and Examples (not in accordance with the present invention).

Referring to FIGS. 4 and 5, it may be seen that structures manufactured have spherical structures (not in accordance with the present invention) and egg-yolk structures according to Examples and Comparative Examples.

Here, in Example 2-1 and Comparative Example 2-1, when spray pyrolysis was performed through dextrin, combustion occurred several times at high temperature, thereby generating the egg-yolk structures.

In addition, referring to FIG. 6, it was confirmed that the nanostructures of Example 1-2, Example1-3, Example1-4, Example 1-5 (not in accordance with the present invention), and Comparative Example 1-1 have spherical structures with full interiors. As a result of element analysis, it was confirmed that Nb was uniformly distributed throughout the structures in the Nb-doped WO₃ spherical structures. In addition, it was confirmed that the amount of Nb capable being observed on the structure surfaces increased as the concentration of Nb added increases.

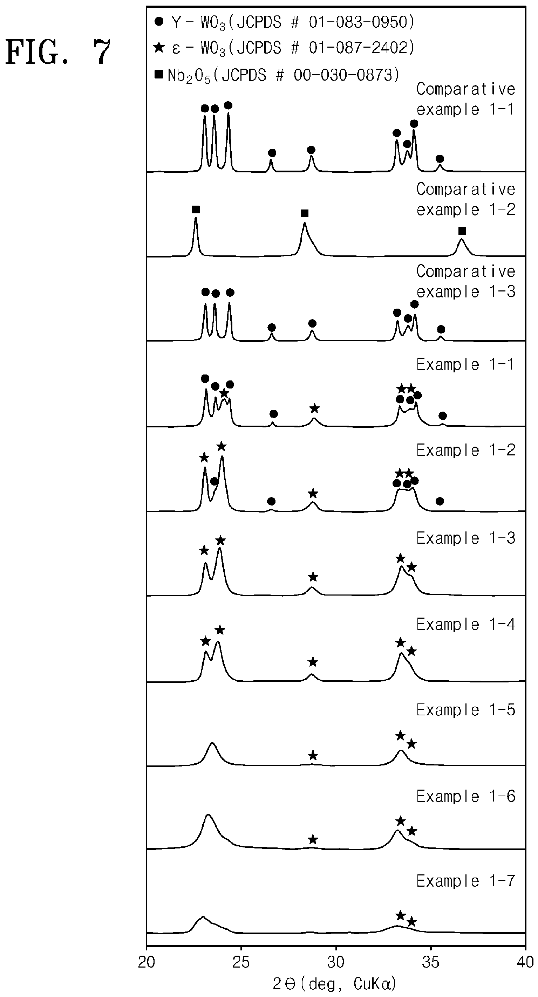

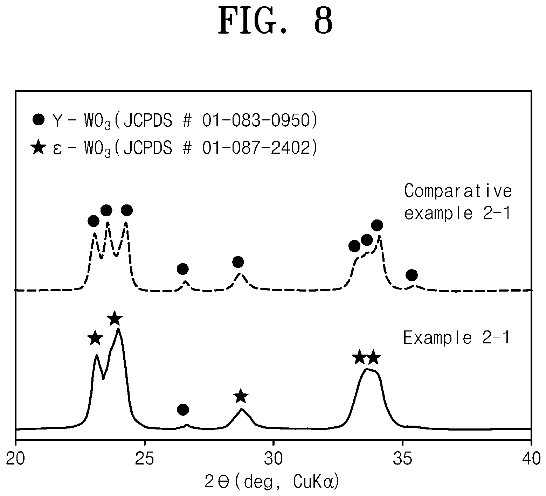

FIGS. 7 and 8 illustrate graphs illustrating results of X-ray diffraction analysis of nanostructures according to Comparative Examples and Examples (the EXAMPLES in FIG. 7 are not according to the present invention, EXAMPLE 2-1 in FIG. 8 is according to the present invention).

First, referring to FIG. 7, in the X-ray diffraction analysis result, it may be confirmed that only a WO₃ γ phase, which was a stable phase at room temperature, existed in Comparative Examples 1-1 and 1-3 in which Nb was not added. It may be confirmed that only Nb₂O₅ was present in Comparative Example 1-2. However, when Nb was added to the spray solution, it may be confirmed that the ferroelectric WO₃ ε phase existed.

It may be seen that when spray pyrolysis was carried out, the Nb and W components were uniformly mixed on a molecular basis, and thus the added Nb mostly replaced W and entered the lattice, resulting in the phase change due to lattice distortion.

In addition, referring to FIG. 8, it may be seen from the results of X-ray diffraction analysis that Comparative Example 2-1, to which Nb was not added, had only the WO₃ γ phase, which is a stable phase at room temperature.

However, it may be seen that the ferroelectric WO₃ ε phase was mixed with the γ phase in Example 2-1 in which Nb was added to the spray solution phase. It may be seen that the added Nb mostly replaced W and entered the lattice, resulting in the phase change due to lattice distortion.

Hereinafter, results of gas sensors manufactured using fine powders synthesized according to Examples and Comparative Examples, as described above, measured at a total of five temperatures of 300 °C, 325 °C, 350 °C, 375 °C and 400 °C will be described.

When a resistance of the sensor became constant in the air in a humidity atmosphere with a relative humidity of 80, the atmosphere was changed to a gas to be tested (acetone, ethanol, ammonia, hydrogen, carbon monoxide, toluene), and when a resistance became constant in the gas, the change in resistance was measured by changing the atmosphere to air again. All of the synthesized sensitizing materials exhibited the characteristics of an n-type oxide semiconductor with a decrease in resistance to a reducing gas. Therefore, the gas sensitivity was defined as S=Ra/Rg (S: gas sensitivity, Ra: element resistance in air, Rg: element resistance in gas). After manufacturing a sensor using the synthesized powder, the gas sensitivity was measured and the sensitivity was compared with other gases to calculate the selectivity.

After manufacturing the sensor using the synthesized fine powder, the gas sensitivity was measured, and the selectivity was calculated by comparing the sensitivity of the other gases.

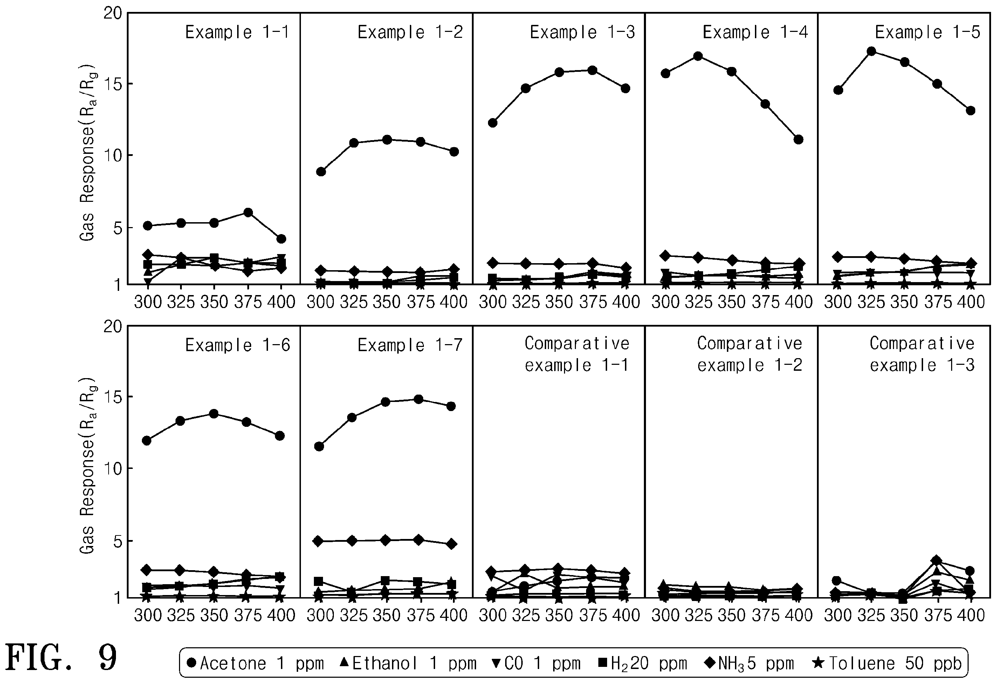

FIG. 9 illustrates graphs of gas responses to 1 ppm acetone, 1 ppm ethanol, 1 ppm carbon monoxide, 20 ppm hydrogen, 5 ppm ammonia and 50 ppb toluene at an operating temperature of 300 °C to 450 °C in gas sensors according to Comparative Examples and Examples (not in accordance with the present invention).

Referring to FIG. 9, when a relative humidity is 80% at operating temperatures 300, 325, 350, 375, and 400 °C, gas sensitivity of Example 1-1, Example 1-2, Example 1-3, Example 1- 4, Example 1-5, Example 1-6, Example 1-7, Comparative Example 1-1, Comparative Example 1-2, and Comparative Example 1-3 to acetone 1 ppm, ethanol 1 ppm, carbon monoxide 1 ppm, 20 ppm of hydrogen, 5 ppm of ammonia, and 50 ppb of toluene are shown, respectively.

A concentration of each biomarker gas was set with reference to a representative concentration from an exhaled air of a person suffering from a disease. In Comparative Example 1-1 to which Nb was not added and Comparative Example 1-2 of pure Nb₂O₅, the sensitivity to the biomarker gas was very small and there was no selectivity to acetone.

In addition, it was confirmed that the sensitivity to the biomarker gas was very small and there was no selectivity to acetone in Comparative Example 1-3 in which the ε phase was not present because Nb was added through the liquid phase process.

However, in Example 1-1, Example 1-2, Example 1-3, Example 1-4, Example 1-5, Example 1-6, and Example 1-7 doped with Nb, it was confirmed that the sensitivity of acetone increased significantly and the sensitivity to other interference gases remained similar.

In particular, in Example 1-3, the sensitivity of acetone at an operating temperature of 375 °C was 16, and the selectivity (=S_{acetone}/S_{interference gas}) was 6.4. That is, the sensitivity of acetone was increased by about 7 times compared to the 2.4 sensitivity of acetone of Comparative Example 1-1 having the pure WO₃, and the selectivity was increased by about 8 times.

This is because, when Nb having a pentavalent valence was doped into WO₃, oxygen vacancy was generated, and the lattice was distorted, thereby causing the phase change from the γ phase stable at the room temperature to the ferroelectric ε phase, it was shown that the sensitivity to acetone, which had a large dipole moment, was significantly increased compared to other interfering gases.

It was shown that Nb doping had a very good effect in increasing the sensitivity and selectivity of acetone of the sensor.

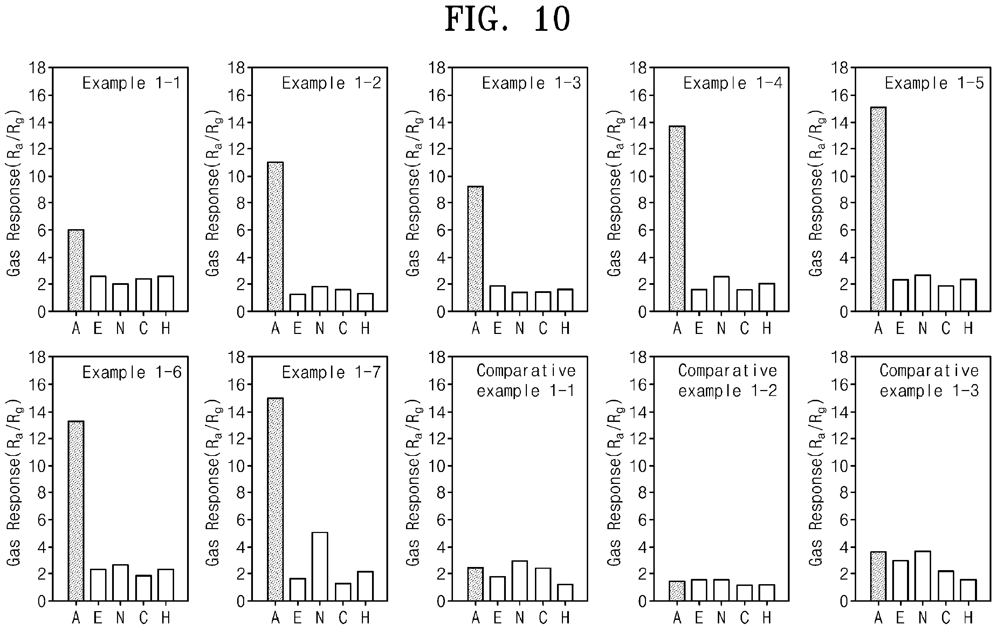

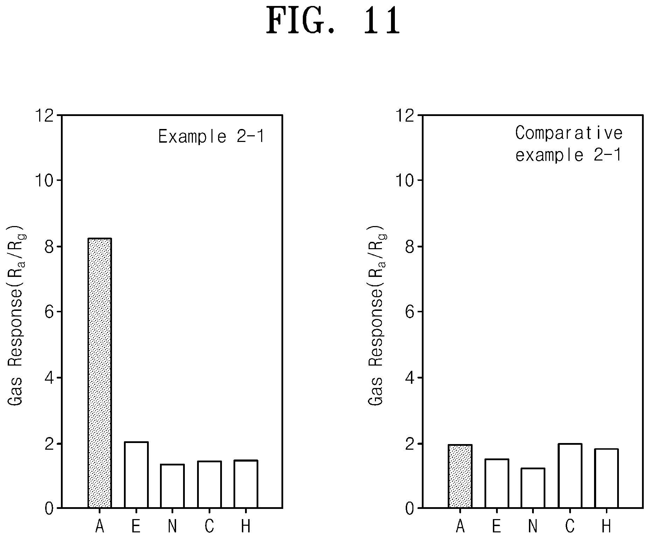

FIGS. 10 and 11 illustrate graphs of gas responses to 1 ppm acetone, 1 ppm ethanol, 1 ppm carbon monoxide, and 1ppm hydrogen at an operating temperature of 375 °C in gas sensors according to Comparative Examples and Examples of the inventive concept.

First, referring to FIG. 10, when the relative humidity was 80% at an operating temperature of 375° C, gas sensitivity of Example 1-1, Example 1-2, Example 1-3, Example 1-4, Example 1-5, Example 1-6, Example 1-7, Comparative Example 1-1, Comparative Example 1-2, and Comparative Example 1-3 to 1 ppm of acetone, 1 ppm of ethanol, 1 ppm of ammonia, 1 ppm of carbon monoxide, and 1 ppm of hydrogen were illustrated.

It may be seen selectively sensitizing characteristics of Example 1-1, Example 1-2, Example 1-3, Example 1-4, Example 1-5, Example 1-6, and Example 1-7 to acetone. It may be seen that the highest acetone sensitive characteristics were illustrated in Example 1-3.

Meanwhile, in Comparative Example 1-1, which was pure WO₃ to which Nb was not added, and in Comparative Example 1-2, which was pure Nb₂O₅ to which W was not added, in Comparative Example 1-3 in which Nb was added as a subsequent process, there was no selectivity and low sensitivity to acetone.

That is, in Examples in which the spray pyrolysis solution was prepared by stirring the W precursor and the Nb precursor, the W and Nb components were mixed in a molecular unit to obtain the desired phase ε phase, and thus high sensitivity and high selectivity for acetone could be obtained. However, because Comparative Example 1-1, Comparative Example 1-2, and Comparative Example 1-3 were the materials without ferroelectricity, it may be confirmed that the selectivity and sensitivity to acetone are small.

Further, FIG. 11 illustrates the gas sensitivity to 1 ppm of acetone, 1 ppm of ethanol, 1 ppm of ammonia, 1 ppm of carbon monoxide, and 1 ppm of hydrogen of Example 2-1 and Comparative Example 2-1 when the relative humidity is 80% at an operating temperature of 375° C.

Here, Example 2-1 exhibited sensitizing characteristics for acetone, and Comparative Example 2-1 exhibited no selectivity and very low sensitivity for acetone.

It may be seen that a sensitizing layer exhibiting ferroelectricity is synthesized due to the phase change occurring when Nb is substituted for WO₃, and sensitivity is improved due to attraction between the sensitizing layer and acetone.

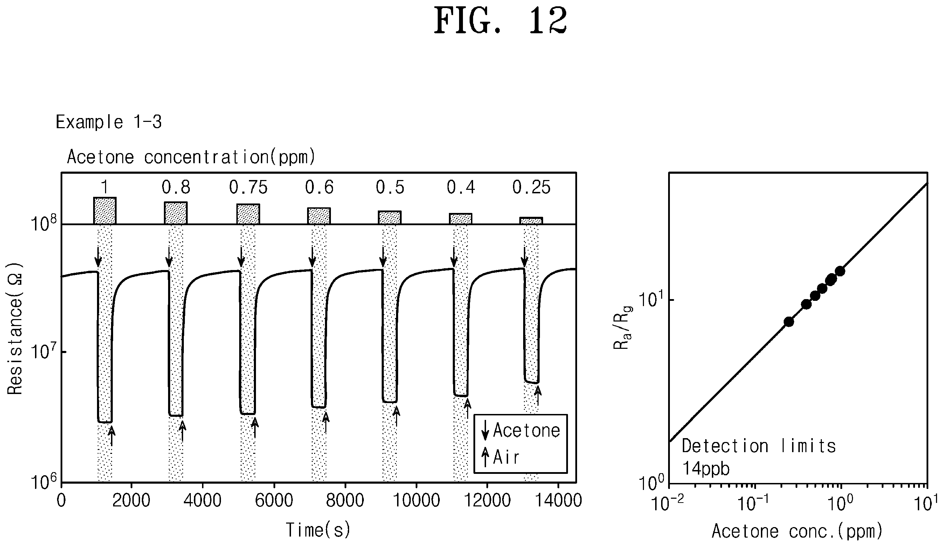

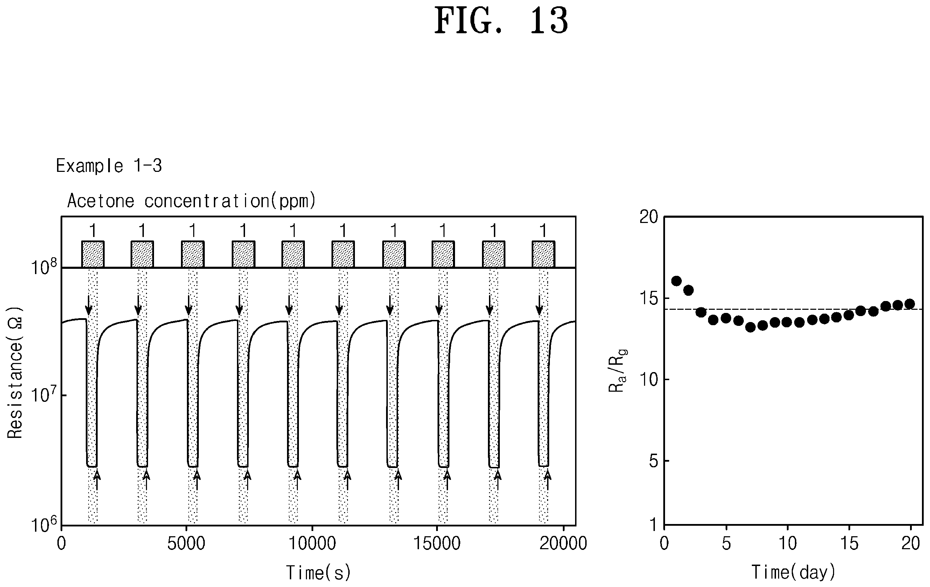

FIG. 12 illustrates graphs of characteristics results for acetone gas concentrations (0.25 to 1 ppm) of gas sensors according to Example 1-3 of the inventive concept at an operating temperature of 375 °C and FIG. 13 illustrates graphs of characteristics results for repetitive acetone gas (1 ppm) of gas sensors according to Example 1-3 (not in accordance with the present invention) at an operating temperature of 375 °C.

First, FIG. 12 illustrates a result of gas sensitizing characteristics and a lower limit of detection calculated therethrough depending on acetone gas concentrations (0.25 to1 ppm) of Example 1-3 when a relative humidity is 80% at an operating temperature of 375° C. It was confirmed that Example 1-3 exhibited high sensitivity even in a humidity atmosphere, and the calculated lower limit of detection was 14 ppb, indicating relatively high sensitivity even at low concentrations.

In addition, FIG. 13 illustrates the results of the repetitive acetone gas (1 ppm) sensitizing characteristics of Example 1-3 when the operating temperature was 375 °C and the relative humidity was 80%. It may be seen that the sensor of Example 1-3 exhibited stable acetone detection characteristics even for repeated measurements of acetone several times and long-term (>20 day) sensor measurements, thereby ensuring reliability.

Accordingly, in the conventional oxide semiconductor type gas sensor, it is difficult to distinguish acetone and other reducing gases by sensitivity, and there is a problem that sensitivity to most gases is lowered, especially in the high humidity atmosphere. Meanwhile, the sensor of the inventive concept may detect acetone with high sensitivity and high selectivity even in the high humidity atmosphere by adjusting the amount of Nb added, and it is possible to measure acetone at the low concentration enough to detect the efficiency of disease diagnosis and ketogenic diet.

According to an embodiment of the inventive concept, the gas sensor using Nb-doped WO₃, may exhibit the low gas sensitivity to the interference gases such as ethanol, ammonia, hydrogen, carbon monoxide, and toluene, and may exhibit the high gas sensitivity and high selectivity to acetone.

Meanwhile, the addition of niobium in the gas sensor changes the phase of tungsten oxide from the stable γ phase at room temperature to the stable ε phase at low temperature to implement the sensitive material having ferroelectricity. Here, unlike the interference gas, acetone having the relatively large dipole moment may be more attracted by the dipole-dipole interaction, and thus acetone may be detected with high sensibility and high selectivity.

Meanwhile, the effects obtainable in the inventive concept are not limited to the above-mentioned effects, and it will understand other effects not mentioned are obvious to those of ordinary skill in the art from the following description.

While the inventive concept has been described with reference to exemplary embodiments, it will be apparent to those skilled in the art that various changes and modifications may be made without departing from the scope of the appended claims. Therefore, it should be understood that the above embodiments are not limiting, but illustrative.

## Claims

1. A resistive gas sensor for selective detection of acetone gas, the gas sensor comprising:
a substrate (140);
a ferroelectric gas sensitizing layer (120; 170) formed of an Nb-doped tungsten oxide, WO₃ nanostructure;
and two electrodes (110, 130; 160, 165) in contact with the gas sensitizing layer (120;170), wherein
the electrodes (160, 165) are formed on an upper surface of the substrate (140), and the gas sensitizing layer (170) is formed thereon,
wherein a concentration ratio Nb/(Nb+W) of niobium, Nb, and tungsten, W, in the gas sensitizing layer is 2at% to 50at%, and
wherein, in the gas sensitizing layer, the Nb-doped WO₃ nanostructure has a egg yolk shaped structure.

2. A method of manufacturing a resistive gas sensor for selective detection of acetone gas, the method comprising:
forming two electrodes (160, 165) on an upper surface of a substrate (140);
forming a Nb-doped WO₃ nanostructure; and
forming a ferroelectric gas sensitizing layer (120; 170) of the Nb-doped WO₃ nanostructure,
wherein the forming of the Nb-doped WO₃ nanostructure includes:
preparing (S10) a mixed solution containing niobium, Nb, and tungsten oxide, WO₃, so that a concentration ratio Nb(Nb+/W) of niobium, Nb, and tungsten, W, is 2 at% to 50 at%, wherein the mixed solution further includes dextrin, forming (S20) a Nb-doped WO₃ precursor with the mixed solution through ultrasonic spray pyrolysis, and
heating the Nb-doped WO₃ precursor to form the Nb-doped WO₃ nanostructure having an egg-yolk-shaped structure due to the spray pyrolysis being performed through dextrin, and
wherein forming (S30) a gas sensitizing layer (120; 170) comprises mixing the Nb-doped WO₃ nanostructure with an organic binder, and applying the mixed solution on the substrate (140) with the two electrodes (160, 165), and
removing a solvent by heat treatment.

3. The method of claim 2, wherein the applying of the mixed solution on the substrate is performed by at least one of printing, brushing, blade coating, dispensing, and micropipette dropping.

## Patentansprüche

1. Resistiver Gassensor zum selektiven Erfassen von Acetongas, wobei der Gassensor Folgendes umfasst:
ein Substrat (140);
eine ferroelektrische Gassensibilisierungsschicht (120; 170), die aus einer Nb-dotierten Wolframoxid-WO₃-Nanostruktur ausgebildet ist;
und zwei Elektroden (110, 130; 160, 165), die mit der Gassensibilisierungsschicht (120; 170) in Kontakt stehen, wobei
die Elektroden (160, 165) auf einer oberen Oberfläche des Substrats (140) ausgebildet sind und die Gassensibilisierungsschicht (170) darauf ausgebildet ist,
wobei ein Konzentrationsverhältnis Nb/(Nb+W) von Niob, Nb, und Wolfram, W, in der Gassensibilisierungsschicht 2at% bis 50at% beträgt, und
wobei in der Gassensibilisierungsschicht die Nb-dotierte WO₃-Nanostruktur eine eigelbförmige Struktur aufweist.

2. Verfahren zur Herstellung eines resistiven Gassensors zum selektiven Erfassen von Acetongas, wobei das Verfahren Folgendes umfasst:
Ausbilden von zwei Elektroden (160, 165) auf einer oberen Oberfläche eines Substrats (140);
Ausbilden einer Nb-dotierten WO₃-Nanostruktur; und
Ausbilden einer ferroelektrischen Gassensibilisierungsschicht (120; 170) aus der Nb-dotierten WO₃-Nanostruktur,
wobei das Ausbilden der Nb-dotierten WO₃-Nanostruktur Folgendes umfasst:
Herstellen (S10) einer gemischten Lösung, die Niob, Nb, und Wolframoxid, WO₃, enthält, so dass ein Konzentrationsverhältnis Nb(Nb+/W) von Niob, Nb, und Wolfram, W, 2at% bis 50at% beträgt, wobei die gemischte Lösung ferner Dextrin enthält,
Ausbilden (S20) eines Nb-dotierten WO₃-Vorläufers mit der gemischten Lösung durch Ultraschall-Sprühpyrolyse und
Erhitzen des Nb-dotierten WO₃-Vorläufers, um die Nb-dotierte WO₃-Nanostruktur aufweisend eine eigelbförmige Struktur auszubilden, die aufgrund der durch Dextrin durchgeführte Sprühpyrolyse entsteht, und
wobei das Ausbilden (S30) einer Gassensibilisierungsschicht (120; 170) das Mischen der Nb-dotierten WO₃-Nanostruktur mit einem organischen Bindemittel und das Aufbringen der gemischten Lösung auf das Substrat (140) mit den beiden Elektroden (160, 165) umfasst, und
Entfernen eines Lösungsmittels durch Wärmebehandlung.

3. Verfahren gemäß Anspruch 2, wobei das Aufbringen der gemischten Lösung auf das Substrat durch mindestens eines der folgenden Verfahren erfolgt: Drucken, Streichen, Rakeln, Dosieren und Tropfen mit einer Mikropipette.

## Revendications

1. Capteur de gaz résistif pour la détection sélective de gaz acétone, le capteur de gaz comprenant :
un substrat (140) ;
une couche sensibilisatrice de gaz ferroélectrique (120 ; 170) formée d'une nanostructure d'oxyde de tungstène dopé au Nb, WO₃;
et deux électrodes (110, 130; 160, 165) en contact avec la couche sensibilisatrice de gaz (120 ; 170), dans lequel
les électrodes (160, 165) sont formées sur une surface supérieure du substrat (140), et la couche sensibilisatrice de gaz (170) est formée sur celles-ci,
dans laquelle un rapport de concentration Nb/(Nb+W) du niobium, Nb, et du tungstène, W, dans la couche sensibilisatrice de gaz est compris entre 2 at% et 50 at%, et
dans laquelle, dans la couche sensibilisatrice de gaz, la nanostructure WO₃ dopée au Nb a une structure en forme de jaune d'œuf.

2. Procédé de fabrication d'un capteur de gaz résistif pour la détection sélective de gaz acétone, le procédé comprenant :
la formation de deux électrodes (160, 165) sur une surface supérieure d'un substrat (140) ;
la formation d'une nanostructure de WO₃ dopé au Nb ; et
la formation d'une couche sensibilisatrice de gaz ferroélectrique (120 ; 170) de la nanostructure de WO₃ dopée au Nb,
dans lequel la formation de la nanostructure de WO₃ dopée au Nb comprend :
la préparation (S10) d'une solution mixte contenant du niobium, Nb, et de l'oxyde de tungstène, WO₃, de telle sorte qu'un rapport de concentration Nb(Nb+/W) entre le niobium, Nb, et le tungstène, W, soit compris entre 2 at% et 50 at%, la solution mixte comprenant en outre de la dextrine,
la formation (S20) d'un précurseur de WO₃ dopé au Nb à partir de la solution mixte par pyrolyse par pulvérisation ultrasonique, et
chauffer le précurseur WO₃ dopé au Nb pour former la nanostructure WO₃ dopée au Nb ayant une structure en forme de jaune d'œuf grâce à la pyrolyse par pulvérisation effectuée par de la dextrine, et
dans lequel la formation (S30) d'une couche sensibilisatrice de gaz (120 ; 170) comprend le mélange de la nanostructure de WO₃ dopée au Nb avec un liant organique, et l'application de la solution mixte sur le substrat (140) avec les deux électrodes (160, 165), et
éliminer un solvant par traitement thermique.

3. Procédé selon la revendication 2, dans lequel l'application de la solution mixte sur le substrat est réalisée par au moins l'un des procédés suivants :
impression, brossage, revêtement à lame, distribution et gouttelettes à la micropipette.
